# EUROPÄISCHE PATENTSCHRIFT

(11) **EP 0 586 488 B1**
(45) Veröffentlichungstag und Bekanntmachung des Hinweises auf die Patenterteilung: **03.05.1995**
(21) Anmeldenummer: 92911421.3
(22) Anmeldetag: 05.06.1992
(51) Int. Cl.: A61K 9/00, A61K 31/60

(54) **PHARMAZEUTISCHE KLISTIERZUBEREITUNG**
PHARMACEUTICAL ENEMA PREPARATION
PREPARATION PHARMACEUTIQUE POUR CLYSTERE

(30) Priorität: 07.06.1991 CH 1690/91; 05.07.1991 DE 4122337
(43) Veröffentlichungstag der Anmeldung: 16.03.1994
(73) Patentinhaber: Byk Gulden Lomberg Chemische Fabrik GmbH, D-78467 Konstanz (DE); Byk Nederland BV, NL-1160 AB Zwanenburg (NL)
(72) Erfinder: WITTEBROOD, Adrianus J., NL-1991 HB Velserbroek (NL); DE JONG, Adrianus P., NL-1985 EW Driehuis (NL); BRON, Jan, NL-3381 KZ Giessenburg (NL)
(74) Vertreter: Rupp, Herbert, Dr.
(86) Internationale Anmeldenummer: EP9201274
(87) Internationale Veröffentlichungsnummer: WO9221324

(56) Entgegenhaltungen:
- EP-A- 0 291 159
- EP-A- 0 395 329
- EP-A- 0 398 207
- FR-A- 2 647 344
- US-A- 4 657 900

## Beschreibung

### Technisches Gebiet

Die Erfindung betrifft eine Klistierzubereitung für 5-Aminosalicylsäure.

### Stand der Technik

5-Aminosalicylsäure (5-ASA) ist ein bekannter Wirkstoff, der vor allem zur Behandlung entzündlicher Darmerkrankungen, wie Colitis ulcerosa, eingesetzt wird. Besonders günstig ist die Anwendung von 5-ASA als Klistierzubereitung, da hiermit das wirksame Prinzip direkt an den Ort der krankhaften Veränderungen gebracht werden kann. Bei diesen Zubereitungen bereitet allerdings die bekannte chemische Instabilität von 5-ASA in Lösungen oder Suspensionen erhebliche Schwierigkeiten. In der Vergangenheit wurden verschiedene Wege beschritten, um vor allem die Licht- und Sauerstoffempfindlichkeit von 5-ASA in den Griff zu bekommen.

So wird in der US-PS 4657900 eine Klistierzubereitung vorgeschlagen, bei der hochreine 5-ASA als wäßrige Suspension vorliegt, die nach Herstellung unter Sauerstoffausschluß und Zusatz von Bisulfit als Antioxidans in einem opaken Polyethylen-Rektalapplikator versiegelt wird, der seinerseits in einem Polyester/Aluminiumfolie/Polyethylen-Beutel hitzeversiegelt wird.

Aus der US-PS 4664256 ist eine sehr ähnliche Verabreichungsform bekannt, in der außer Bisulfit als Antioxidans noch ein Chelatbildner, wie z.B. Ethylendiamintetraacetat (EDTA) enthalten ist.

Das Problem der Instabilität bei Klistierzubereitungen wird durch diese Maßnahmen anscheinend leidlich gelöst.

Abgesehen von der aufwendigen Verpackung bei den 5-ASA-Klistierzubereitungen nach dem Stand der Technik zeigen die Zubereitungen jedoch Probleme was die Resuspendierbarkeit der 5-ASA-Suspension vor dem Gebrauch angeht. Die bekannten Zubereitungen müssen nämlich vor Gebrauch sehr kräftig und ausdauernd geschüttelt werden, um eine für die Anwendung wünschenswerte homogene Verteilung des Wirkstoffs zu erreichen. Es versteht sich von selbst, daß diese mühsame Resuspendierung insbesondere von gebrechlichen Patienten nur unzureichend bewältigt wird und insgesamt für die Patienten-Compliance nicht förderlich ist.

### Beschreibung der Erfindung

Es wurde nun überraschenderweise gefunden, daS sich 5-ASA-Klistierzubereitungen nach dem Stand der Technik durch den Zusatz von Titandioxid in mehrfacher Hinsicht verbessern lassen. Die Licht und Oxidationsstabilität von 5-ASA-Suspensionen erhöht sich durch diesen erfindungssgemäßen Zusatz derart, daß es nicht mehr notwendig ist, die 5-ASA-Suspension in einem doppelwandigen Behälter zur Verfügung zu stellen. Als weiteren Vorteil ergibt sich, daß die Stabilität der Suspension so erhöht wird, daß das lästige Resuspendieren vor der Anwendung durch Schütteln entfallen kann.

Gegenstand der Erfindung ist daher eine wäßrige 5-Aminosalicylsäure-Klistiersuspensionszubereitung, die dadurch gekennzeichnet ist, daß neben den üblichen Hilfsstoffen Titandioxid enthalten ist.

Unter üblichen Hilfsstoffen werden solche Hilfsstoffe verstanden, die für Suspensionen üblicherweise eingesetzt werden und solche, die zur Stabilisierung von 5-ASA-Suspensionen gebräuchlich sind. Zur ersten Gruppe zählen z.B. die üblichen viskositätserhöhenden Stoffe, Konservierungsmittel, wie z.B. Benzoesäure, den pH-Wert regulierende Stoffe, wie Puffer, Chelatbildner, wie z.B. EDTA, und Antioxidantien, wie insbesondere die bei 5-ASA-Zubereitungen üblichen Bisulfite.

Titandioxid wird erfindungsgemäß in einer Menge von 0,1 bis 3, vorzugsweise 0,1 bis 1 und insbesondere 0,5 Gew.-% zugefügt.

5-ASA ist in den erfindungsgemäßen Suspensionen in einer Menge von 0,5 bis 10, vorzugsweise 2 bis 6 und insbesondere etwa 4 Gew.-% enthalten.

Bisulfite als Antioxidantien sind in den erfindungsgemäßen Suspensionen in einer Menge von 0,05 bis 0,5, vorzugsweise 0,1 bis 0,2 und insbesondere etwa 0,15 Gew.-% enthalten.

Viskositätserhöhende Stoffe sind in den erfindungsgemäßen Suspensionen in einer Menge von 0,05 bis 2, vorzugsweise 0,1 bis 1 und insbesondere etwa 0,5 Gew.-% enthalten.

Chelatbildner sind in der erfindungsgemäßen Suspension in einer Menge von 0,01 bis 0,5, vorzugsweise 0,05 bis 0,2 und insbesondere etwa 0,1 Gew.% enthalten.

Die Herstellung der erfindungsgemäßen Suspension erfolgt nach den aus dem Stand der Technik bekannten Verfahren unter Sauerstoffausschluß. Die fertige Suspension wird dann unter Sauerstoffausschluß in übliche flexible Klistierapplikatoren abgefüllt, die vorzugsweise aus opakem Kunststoff hergestellt werden.

### Herstellungsbeispiel

Nach den üblichen Verfahren wird eine Suspension hergestellt und in Klistierbehälter abgefüllt, die pro 50 g Klistierpackung folgende Bestandteile enthält:

| | |
|---|---|
| 5-Aminosalicylsäure | 2,000 g |
| Natriumdihydrogenphosphat x 2H₂O | 0,500 g |
| Natriumedetat | 0,050 g |
| Benzoesäure | 0,100 g |
| Titandioxid E 171 | 0,250 g |
| Natriumdisulfit | 0,075 g |
| Xanthan Gummi | 0,250 g |
| Natriumhydroxid-Lösung 10 M | (bis pH 4,0) |
| Gereinigtes Wasser | bis 50,00 g |

## Patentansprüche (Patentansprüche für folgende(n) Vertragsstaat(en): AT, BE, CH, LI, DE, DK, FR, GB, IT, LU, MC, NL, SE, PT)

1. Wäßrige 5-Aminosalicylsäure-Klistiersuspensionszubereitung, dadurch gekennzeichnet, daß neben üblichen Hilfsstoffen Titandioxid enthalten ist.

2. Zubereitung nach Anspruch 1, dadurch gekennzeichnet, daß 0,1 bis 3 Gew.-% Titandioxid enthalten sind.

3. Zubereitung nach Anspruch 1, dadurch gekennzeichnet, daß 5-Aminosalicylsäure in einer Menge von 0,5 bis 10 Gew.% enthalten ist.

4. Zubereitung nach Anspruch 1, dadurch gekennzeichnet, daß als Hilfsstoffe viskositätserhöhende Stoffe, Konservierungsstoffe, Chelatbildner, Puffer und Antioxidantien enthalten sind.

## Patentansprüche (Patentansprüche für folgende(n) Vertragsstaat(en): ES, GR)

1. Verfahren zur Herstellung einer wäßrigen 5-Aminosalicylsäure-Klistiersuspensionszubereitung, dadurch gekennzeichnet, daß man 5-Aminosalicylsäure zusammen mit üblichen Hilfsstoffen und Titandioxid in Wasser suspendiert.

2. Verfahren nach Anspruch 1, dadurch gekennzeichnet, daß man 0,1 bis 3 Gew.-% Titandioxid einarbeitet.

3. Verfahren nach Anspruch 1, dadurch gekennzeichnet, daß man 5-Aminosalicylsäure in einer Menge von 0,5 bis 10 Gew.% einarbeitet.

4. Verfahren nach Anspruch 1, dadurch gekennzeichnet, daß man als Hilfsstoffe viskositätserhöhende Stoffe, Konservierungsstoffe, Chelatbildner, Puffer und Antioxidantien einarbeitet.

## Claims (Claims for the following Contracting State(s): AT, BE, CH, LI, DE, DK, FR, GB, IT, LU, MC, NL, SE, PT)

1. Aqueous 5-aminosalicylic acid enema suspension preparation, characterised in that titanium dioxide is present in addition to customary auxiliaries.

2. Preparation according to Claim 1, characterised in that 0.1 to 3 % by weight of titanium dioxide is present.

3. Preparation according to Claim 1, characterised in that 5-aminosalicylic acid is present in an amount from 0.5 to 10 % by weight.

4. Preparation according to Claim 1, characterised in that viscosity-enhancing substances, preservatives, chelating agents, buffers and antioxidants are present as auxiliaries.

## Claims (Claims for the following Contracting State(s): ES, GR)

1. Process for the preparation of an aqueous 5-aminosalicylic acid enema suspension, characterized in that 5-aminosalicylic acid is suspended in water with customary auxiliaries and titanium dioxide.

2. Process according to claim 1, characterized in that titanium dioxide is added in an amount from 0.1 to 3 % by weight.

3. Process according to claim 1, characterized in that 5-aminosalycil acid is added in an amount from 0.5 to 10 % by weight.

4. Process according to claim 1, characterized in that viscosity-enhancing substances, preservatives, chelating agents buffers and antioxidants are added as auxiliaries.

## Revendications (Revendications pour l'(les) Etat(s) contractant(s) suivant(s): AT, BE, CH, LI, DE, DK, FR, GB, IT, LU, MC, NL, SE, PT)

1. Préparation de suspension aqueuse pour lavement à base d'acide 5-aminosalicylique, caractérisée en ce qu'elle contient du dioxyde de titane, outre les adjuvants habituels.

2. Préparation suivant la revendication 1, caractérisée en ce qu'elle contient de 0,1 à 3% en poids de dioxyde de titane.

3. Préparation suivant la revendication 1, caractérisée en ce qu'elle contient de l'acide 5-aminosalicylique en une proportion de 0,5 à 10% en poids.

4. Préparation suivant la revendication 1, caractérisée en ce qu'elle contient, à titre d'adjuvants, des substances augmentant la viscosité, des conservateurs, des agents formateurs de chélates, des tampons et des antioxydants.

## Revendications (Revendications pour l'(les) Etat(s) contractant(s) suivant(s): ES, GR)

1. Procédé de réalisation d'une préparation d'une suspension aqueuse pour lavement à base d'acide 5-aminosalicylique, caractérisé en ce que l'on met de l'acide 5-aminosalicylique en même temps que des adjuvants usuels et du dioxyde de titane en suspension dans de l'eau.

2. Procédé suivant la revendication 1, caractérisé en ce que l'on incorpore de 0,1 à 3% en poids de dioxyde de titane.

3. Procédé suivant la revendication 1, caractérisé en ce que l'on incorpore de l'acide 5-aminosalicylique en une proportion de 0,5 à 10% en poids.

4. Procédé suivant la revendication 1, caractérisé en ce que l'on incorpore, à titre d'adjuvants, des produits augmentant la viscosité, des conservateurs, des agents formateurs de chélates, des tampons et des antioxydants.
